# EUROPEAN PATENT APPLICATION

(11) **EP 4 721 711 A1**
(43) Date of publication of application: **08.04.2026**
(21) Application number: 25203026.7
(22) Date of filing: 18.09.2025
(51) Int. Cl.: A61F 7/00, A61H 33/00

(54) **COLD AND HOT PLUNGE SHOWER MANAGEMENT SYSTEMS**

(30) Priority: 02.10.2024 US 202463702278 P; 17.09.2025 US 202519330983
(71) Applicant: Kohler Mira Limited, Cheltenham, Gloucestershire GL52 5EP (GB)
(72) Inventor: BLENKARN, Daniel, Cheltenham, GL52 5EP (GB); HOBBS, Barry, Cheltenham, GL52 5EP (GB); LEA, Ben, Cheltenham, GL52 5EP (GB)
(74) Representative: Barker Brettell LLP

(57) **Abstract**

The present disclosure relates to a shower management system including the ability to provide a user with cold or hot boost therapy sessions. The boost therapy shower management system may include a first water outlet and a second water outlet in fluid communication with a one or more fresh water supplies and one or more optional boost therapy water storage tanks. Following an predetermined time or upon activation by a user, a boost therapy shower session may be administer to a user and cold or hot water may be output through a shower head, wherein the boost therapy water is output from the one or more optional boost therapy water storage tanks at a predetermined temperature for a predetermined period of time.

## Description

### Cross-Reference to Related Applications

The present application claims priority to U.S. Provisional Patent Application No. 63/702,278, filed October 2, 2024, entitled, "COLD AND HOT PLUNGE SHOWER MANAGEMENT SYSTEMS," and U.S. Patent Application No. 19/330,983, filed September 17, 2025, the entire contents of which are hereby incorporated by reference.

### Background

Cold water therapy (also referred to as cold water boost therapy or cryotherapy) and hot boost therapy (e.g., hydrotherapy) may provide users many benefits. Commonly, cold showers are achieved by a variety of methods. As a first example, turning a standard shower's water temperature to cold may use the main water line's cold input. As a second example, a chilled water tank may be plumbed into a shower, and then a user may use an application to turn the chilled water supply on and off.

### Brief Description of Drawings

Fig. 1 is a schematic diagram of an example multi-inlet cold boost therapy shower management system.
Fig. 2 is a schematic diagram of an example multi-inlet hot boost therapy shower management system.
Fig. 3 is a schematic diagram of an example multi-inlet cold and hot boost therapy shower management system.
Fig. 4 is example view of a controller for transitional operation into a cold or warm boost therapy session.
Fig. 5 is an exemplary view of a controller during countdown prior to initiating a boost therapy session.
Fig. 6 is an exemplary view of a controller during a boost therapy session.
Fig. 7 is an exemplary view of a shower head for output of cold or hot shower boost therapy sessions.
Fig. 8 is an exemplary view of a remote-control application for implementing a boost therapy shower management system for cold or hot shower boost therapy sessions.

While the disclosure is susceptible to various modifications and alternative forms, a specific embodiment thereof is shown by way of example in the drawings and will herein be described in detail. It should be understood, however, that the drawings and detailed description presented herein are not intended to limit the disclosure to the particular embodiment disclosed, but to the contrary, the intention is to cover all modifications, equivalents, and alternatives falling within the spirit and scope of the present disclosure as defined by the appended claims.

### Detailed Description

The invention will now be described with reference to the drawing figures, in which like reference numerals refer to like parts throughout. For purposes of clarity in illustrating the characteristics of the present invention, proportional relationships of the elements have not necessarily been maintained in the drawing figures.

The present disclosure relates to a shower management system including the ability to provide a user with cold or hot boost therapy sessions. The boost therapy shower management system may include a first water outlet and a second water outlet in fluid communication with a one or more fresh water supplies and one or more optional boost therapy water storage tanks. Following a predetermined time or upon activation by a user, a boost therapy shower session may be administered to a user, and cold and/or hot water may be output through a shower head. The boost therapy water may be output from the one or more optional boost therapy water storage tanks at a predetermined temperature for a predetermined period of time.

Before turning to the figures, which illustrate exemplary systems involving incorporating cold or hot water plunge systems within shower systems, it should be understood that the present disclosure is not limited to the details or methodology set forth in the description or illustrated in the figures. It should also be understood that the terminology used herein is for the purpose of description only and should not be regarded as limiting.

A boost therapy shower system may include one or more water spray outputs. For example, the spray outputs may include one or more spray heads to output, at least in part, water into a shower space. Spray heads may output fresh, chilled, cold, or freezing water, as may be intended for a cold water boost therapy exercise. In other examples, spray heads may output heated or hot water, as may be intended for a hot water boost therapy exercise. Alternative temperatures of water, as further described herein, may also be used. Spray heads may be mounted anywhere within the shower space. Spray heads may be rotatable or otherwise adjusted to spray water into shower space. Spray heads may be fluidly in communication with a plumbing system to output hot, cold or a combination of water temperatures into shower space.

A boost therapy shower system may include a combination of a mixing valve (which may be a digital mixing valve), an exchange engine, a user interface (UI) or controller, and at least one power supply. The mixing valve and exchange engine may be in fluid communication with plumbing system to facilitate water output into shower space via one or more water inlets. For example, one or more inlets may include a cold water inlet and a hot water inlet. The mixing valve, exchange engine, and UI may be in electrical communication to facilitate control and operation of boost therapy shower.

The mixing valve, exchange engine, and UI may be in electrical communication with the power supply of the boost therapy shower system. The power supply may power the components of the boost therapy shower system, such as for the mixing valve, exchange engine, UI, motors, sensors, pumps, and other electrical components within boost therapy shower system, as described herein. The UI may be programed or otherwise in communication with the mixing valve, exchange engine, power supply, and other various components of the boost therapy shower system as generally described herein to facilitate control of water output and boost water therapy exercises. The UI may be programmed to facilitate and control water temperature output, water flow, type of water spray, transitioning between typical shower output and boost water therapy facilitation, etc. It should be understood, while example control and output variations are described herein, the examples are not an exhausted list and other combinations of water flow, control, etc. are contemplated.

The boost therapy shower system may include a water supply, such as a cold water supply or hot water supply or a combination thereof, which may be a water supply from a grid to supply fresh water through individual water supply lines. A cold water supply through the cold water inlet may be in fluid communication with the spray head via a piping and valve network as described and shown herein. A hot water supply through the hot water inlet may be in fluid communication with the spray head via a piping and valve network as described and shown herein.

The cold and hot water supplies may be coupled with, and respective water may flow through, a mixing and control unit, such as a digital mixing valve. The fresh cold water inlet and hot water inlet may be provided individually or mixed as a blended output to enable the water from the two water inlets to reach a desired temperature and pressure. Such desired pressure and temperature may be requested by an individual taking a shower, via control from controller or UI. The mixing valve may be mechanically or electronically controlled. Temperature and flow control of water from the cold water inlet or hot water inlet will be understood by one skilled in the art.

The blended outlet, from the digital mixing valve, may then flow through a portion of the designated plumbing system and to the shower head. This process may be considered standard operations of a shower for a user, in which pressure, temperature, spray type, etc. may be controlled through the controller or UI and optional manipulation of the spray head.

An alternative mode of operation may be desired and activated by a user, via the controller or UI. Upon activation, a user may initiate a cold or hot water boost therapy session, as described herein. Prior to activation, cold water may be received from cold water inlet, into mixing valve, and flow through a secondary outlet, among a portion of the plumbing system, and into the exchange engine. The exchange engine may include a tank, chiller unit, heat exchanger, and pump. Cold water may be received and stored into the chilled tank for later use, for example, during a cold boost therapy session. The tank may store, chill, or heat received water from the cold water inlet or hot water inlet. The chiller unit or heat exchanger, which may facilitate cooling or heating of the received water in the stored water in the tank to the desired temperature, may be controlled and indicated by the user via controller or UI. The desired temperature for cold or hot boost water therapy may be predetermined for automatic heating and cooling of the received water in tank. Upon activation of the cold or hot water boost therapy session, via the controller or UI, the pump may pump the water from tank through a chilled or heated outlet to be output through the spray head. This alternative mode of operation may be considered by a user to be a session of direct cold or hot boost therapy.

The cold or hot boost therapy session may be set on a timer to facilitate a session for a predetermined period of time. The cold or hot boost therapy session may be set to pump the entire supply of water within the tank, or any additional or alternative timing measures in which the user configures.

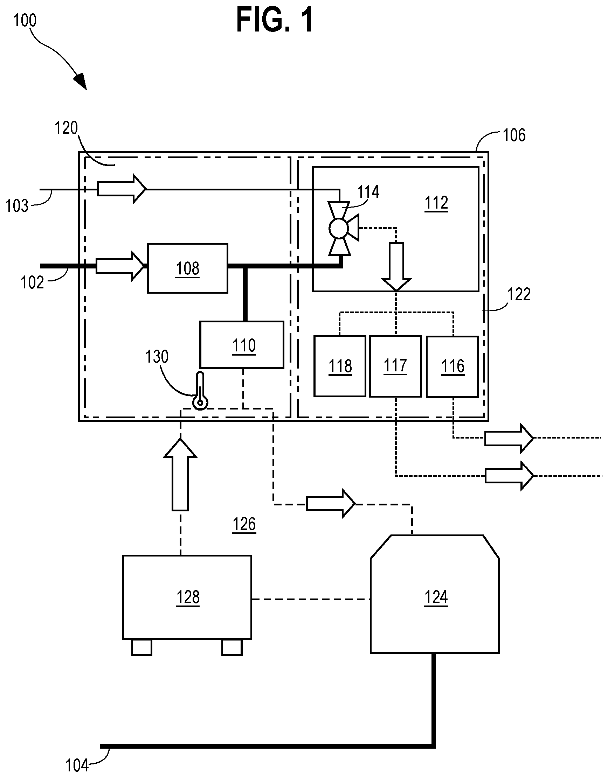

Turning first to Fig. 1, the shower management system 100 may include the components necessary to facilitate water output and distribution of freshwater, cold boost therapy sessions, hot boost therapy sessions, etc. To assist in facilitating cold boost therapy, the shower management system 100 may include multiple inlets to facilitate normal shower usage and cold boost shower sessions. The shower management system 100 may be in fluid communication with a cold freshwater inlet 102 and a hot freshwater inlet 103, as well as a second cold main water inlet 104. The cold freshwater inlet 102 and hot freshwater inlet 103 may feed freshwater into a digital mixing valve 106 during use of a shower. The digital mixing valve 106 may include one or more solenoid valves to aid in facilitation of control of water flow, for example, for normal shower usage and for cold boost therapy sessions.

One or more solenoid valves may include a cold solenoid 108 and a chilled solenoid 110, which may be in fluid communication with the cold freshwater inlet 102. During normal use of a shower by a user, water may flow from the cold freshwater inlet 102 and hot freshwater inlet 103 into a water mixing valve 112. Water temperature may be controlled by a hot and cold stepper 114, which may be determined by a user via a UI as similarly described above. The water output, which may be a controlled and determined temperature, may be output to one or more outlets, including outlets 116, 117, 118, etc. Under normal shower operating conditions, the controlled and determined temperature may be output through a first outlet 116. The digital mixing valve 106 may include an inlet management unit 120 and a digital water control unit 122, wherein the inlet management unit 120 may include, at least in part, the solenoid valves 108, 110, and the digital water control unit 122 may include the hot and cold stepper 114 to facilitate water output to outlets 116, 117, 118, etc.

The cold main freshwater inlet 104 may be in fluid communication with a chilled storage tank 124 and may be activated, either automatically or manually, to input cold water into the storage tank 124 for later use. The cold water reserved in the storage tank 124 may be intended for a cold boost therapy session. Upon activation of a cold water boost therapy session, cold water may be pumped through the shower management system 100, which may be facilitated into and through water mixing valve 112 and may output through an alternative outlet 117.

The storage tank 124 may circulate through a recirculating loop as opposed to a direct feed via recirculating plumbing 126. A filling, distributing, or recirculating system may provide storage of cold or hot boost therapy water, dispensing of said water and the retention of the temperature of said water. Whether a boost therapy session has been activated or retention of temperature has initiated or continued, water may be pumped from the chilled storage tank 124 and through a chiller 128, via the recirculating plumbing 126. The chilled water may continue through the digital mixing valve 106, which may comprise a thermistor 130. The thermistor 130 may analyze the temperature of the water from the storage tank 124. Should the temperature of the water analyzed by the thermistor 130 be too hot or too cold, the thermistor 130 may communicate with the chiller 128 for proper adjustment and, thus, recirculation may continue to achieve a desired temperature of water for a boost therapy session. An integrated thermistor may provide the boost therapy shower management system 100 with an integrated chilled or heated monitoring system. The temperature of the water within the storage tank 124 may be predetermined or manually indicated by a user, via controller or UI. Upon activation of a boost therapy session, water from the storage tank 124 may flow through the recirculating plumbing 126, through digital mixing valve 106 and output through a secondary output 117, as depicted. The chilled solenoid 110 may control the input or output of the cold freshwater inlet 102 into the storage tank 124 or output of water through the output 117, which may facilitate optional or additional filling of the storage tank 124. The desired temperature and pressure may be analyzed by the thermistor 130 and maintained by the chiller 128 and hot and cold steppers 114 for output to a user. Variations of the storage tank 124, chiller 128, heat exchanger, pump, and any other components may be incorporated to facilitate the storage and output of water for cold or hot boost therapy shower sessions.

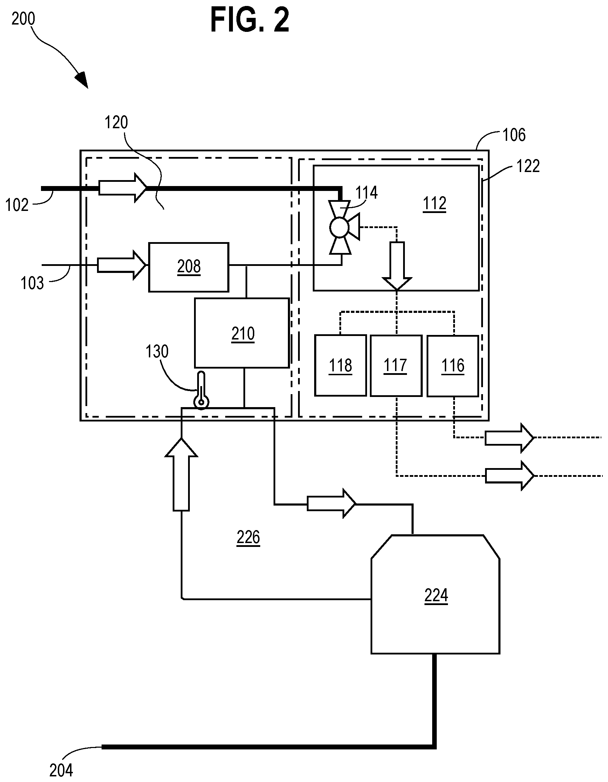

Turning to Fig. 2, a shower management system 200 may include the same or similar components necessary to facilitate water output and distribution of freshwater, cold boost therapy sessions, and in this example, hot boost therapy sessions. To assist in facilitating hot boost therapy, the shower management system 200 may include multiple inlets to facilitate normal shower usage and hot boost shower sessions. The shower management system 200 may be in fluid communication with a cold freshwater inlet 102, a hot freshwater inlet 103, and a second mains water inlet 204. The multi-inlet hot boost therapy shower management system 200 may include the same or similar components and configurations as described herein. The hot boost therapy shower management system 200 may include minor variations to the cold boost therapy shower management system 100 described above with reference to Fig. 1.

The multi-inlet hot boost therapy shower management system 200 may interchange the cold solenoid 108 and chilled solenoid 110 (not illustrated in Fig. 2) with a hot solenoid 208 and secondary hot solenoid 210 (fluidly coupled with hot freshwater inlet 103) in order to facilitate the flow of hot water or a hot water boost therapy session. The water output, which may be a controlled and determined temperature, may be output to one or more outlets, including outlets 116, 117, 118, etc. A digital mixing valve 106 may include an inlet management unit 120 and a digital water control unit 122, wherein the inlet management unit 120 may include, at least in part, the solenoid valves 208, 210, and the digital water control unit 122 may include the hot and cold stepper 114 to facilitate water output to outlets 116, 117, 118, etc.

A hot main water inlet 204 may be in fluid communication with a hot storage tank 224 and may be activated, either automatically or manually, to input main water into a storage tank 224 for later use. Hot water stored in the storage tank 224 may be intended for a hot boost therapy session. Upon activation of a hot water boost therapy session, as described herein, hot water may be pumped through the shower management system 200, which may be facilitated into and through the water mixing valve 112 and output through an alternative outlet, for example, outlet 117.

The storage tank 224 may circulate through a recirculating loop, as opposed to a direct feed, via recirculating plumbing 226. A filling, distributing, or recirculating system may provide storage of hot boost therapy water, dispensing of said water and the retention of temperature of said water. Whether a boost therapy session has been activated or retention of temperature has initiated or continued, water may be pumped from the hot storage tank 224, via the recirculating plumbing 226. The hot water may continue through the digital mixing valve 106, which may comprise a thermistor 130. The thermistor 130 may analyze the temperature of the water from the storage tank 224. Should the temperature of the water analyzed by the thermistor 130 be too hot or too cold, the thermistor 130 may communicate with a hot and cold stepper 114 for proper adjustment and, thus, recirculation may continue to achieve a desired temperature of water for a boost therapy session. The temperature of the water within the storage tank 224 may be predetermined or manually indicated by a user, via controller or UI.

Upon activation of a boost therapy session, water from the storage tank 224 may flow through recirculating plumbing 226, through the digital mixing valve 106 and output through a secondary output 117, as depicted. The secondary hot solenoid 210 may control the input or output of the hot freshwater inlet 103 into storage tank 224 for optional or additional filling of the storage tank 224 and output of water through the output 117. The desired temperature and pressure may be analyzed by the thermistor 130 and maintained by the cold freshwater inlet 102 and hot freshwater inlet 103 and hot and cold steppers 114, within digital mixing valve 106 for output to a user. The storage tank 224 may include, at least in part, a hot storage tank, heat exchange, pump, and any other components to facilitate storage and output of water for a hot boost therapy shower session. The multi-inlet hot boost therapy shower management system 200 may include a heater unit in fluid communication with recirculating plumbing 226 to assist in heating of the water stored in hot storage tank 224. The heater unit may assist in heating the water for an activated hot boost therapy or for maintaining a desired temperature of water in the hot storage tank 224. The temperature of the stored water may be maintained through the recirculating plumbing 226 and thermistor 130 as depicted.

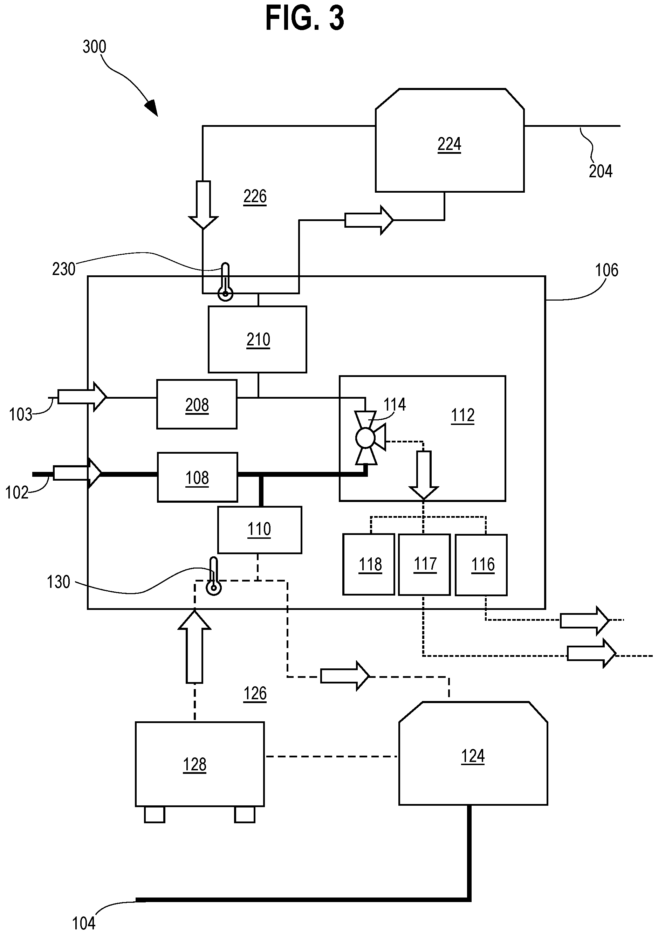

Turning now to Fig. 3, a shower management system 300 may include the same or similar components necessary to facilitate selective water output and distribution of freshwater, cold boost therapy sessions, and hot boost therapy sessions. To assist in facilitating cold and/or hot boost therapy, the shower management system 300 may include multiple inlets to facilitate normal shower usage, cold boost therapy sessions, hot boost shower sessions, etc. The multi-inlet cold or hot boost therapy shower management system 300 may include a cold freshwater inlet 102 and a hot freshwater inlet 103, cold main inlet 104, and an additional main water inlet 204, which may be in fluid communication with shower management system 300.

The shower management system 300 may include a combination of boost therapy shower management variations as described above. The multi-inlet cold or hot boost therapy shower management system 300 may integrate both cold boost therapy management and hot boost therapy management configurations. The cold solenoid 108 and chilled solenoid 110 (fluidly coupled with cold freshwater inlet 102) and the hot solenoid 208 and secondary hot solenoid 210 (fluidly coupled with hot freshwater inlet 103) may be integrated into a digital mixing valve 106 to facilitate the flow of water, a cold hot water boost therapy session, or a hot water boost therapy session. Both a chilled storage tank 124 and a hot storage tank 224 may be integrated to store cold or hot water and facilitate cold or hot therapy sessions. Recirculating loops may be integrated via recirculating plumbing 126, 226, respectively, to maintain desired temperatures for each storage tank 124, 224, respectively, by monitoring the temperatures via thermistors 130, 230, respectively. The thermistor 130 may communicate with a chiller 128 for proper adjustment of the temperature within recirculating plumbing 126. One or more storage tanks 124, 224 may be a direct feed through digital mixing valve 106 and though one or more outputs 116, 117, 118, etc. Each output 116, 117, 118 may have a designated, fluidly coupled output corresponding to one of the normal shower operating conditions, the chilled storage tank 124, and hot storage tank 224.

During normal use of a shower by a user, water may flow from the cold freshwater inlet 102 and hot freshwater inlet 103 into a water mixing valve 112. Water temperature may be controlled by a hot and cold stepper 114, which may be determined by a user via a UI as similarly described above. Under normal operating shower conditions, the output 116 may output a desired temperature, pressure, etc. of water to a user. Under a cold therapy session, the output 117 may output the water from the chilled storage tank 124 at the indicated or predetermined temperature, pressure, and for period of time to a user. Under a hot therapy session, the output 118 may output the water from the hot storage tank 224 at the indicated or predetermined temperature, pressure, and for a period of time to a user. It will be understood that replication of the described above configurations and integration of both cold and hot therapy session sessions have not been reiterated for ease of reference.

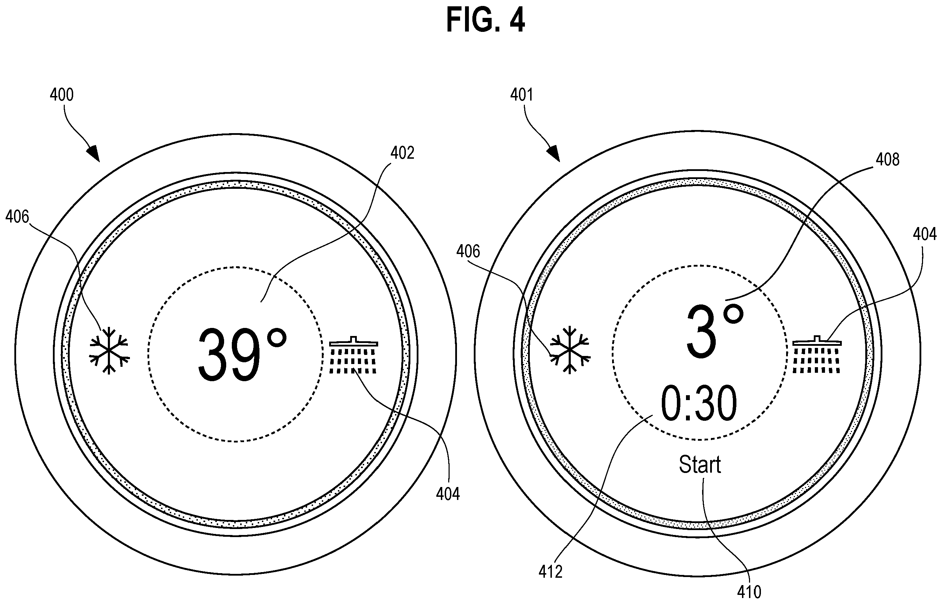

As illustrated in Fig. 4, during normal shower operations, a first display 400 may be depicted on a controller or UI. The indicated and desired temperature of the water output may be indicated by a temperature display 402. Temperature of water output may be optionally controlled and changed by rotational control, digital or mechanical buttons, voice activations, etc., integrated into the controller, which would be updated and shown on the temperature display 402. The controller may include optional features for selection, such as activating normal shower operations 404 and inducing a cold or hot boost therapy session 406. During use, a user may select normal shower operations 404 to commence normal shower usage. Normal shower usage 404 may be set as the default usage for the user. A user may manually activate a boost therapy session by selecting and activating the cold or hot boost therapy session 406.

A second display 401 (which may be associated with a boost shower therapy session) may be displayed prior to, during, and following boost shower therapy session operations on the controller or UI. Optional selection of a cold or hot boost therapy session may initiate said therapy sessions. During a boost therapy session, as described herein, the display of the controller may change, as exemplarily depicted by second display 401. The second display 401 may continue to depict optional activation and deactivation selection of normal shower operations 404 and boost therapy sessions 406. The previous temperature display 402 may change to a temperature display 408 indicating the temperature of said activated boost therapy session. Upon selection by the user, the boost therapy session may be automatically initiated. The user may be presented with an optional "start" selection 410 to manually initiate the boost therapy session. A timer 412, for example, a countdown timer, may also be displayed on the second display 401 during the boost therapy session. During the boost therapy session, a countdown timer 412 may display the amount of time remaining for the boost therapy session.

It should be understood that, while only two optional selections are indicated, additional or alternative selections may be incorporated into the controller or UI. For example, when a combination of cold and hot boost therapy sessions is available, additional user selections may be made available into the controller to activate and deactivate such functions. Additionally, while it may not be explicitly indicated, selection of normal shower usage 404, cold boost therapy sessions 406, hot boost therapy sessions, etc. may be indicated by a physical press button, a digital touch indication, voice activation, etc.

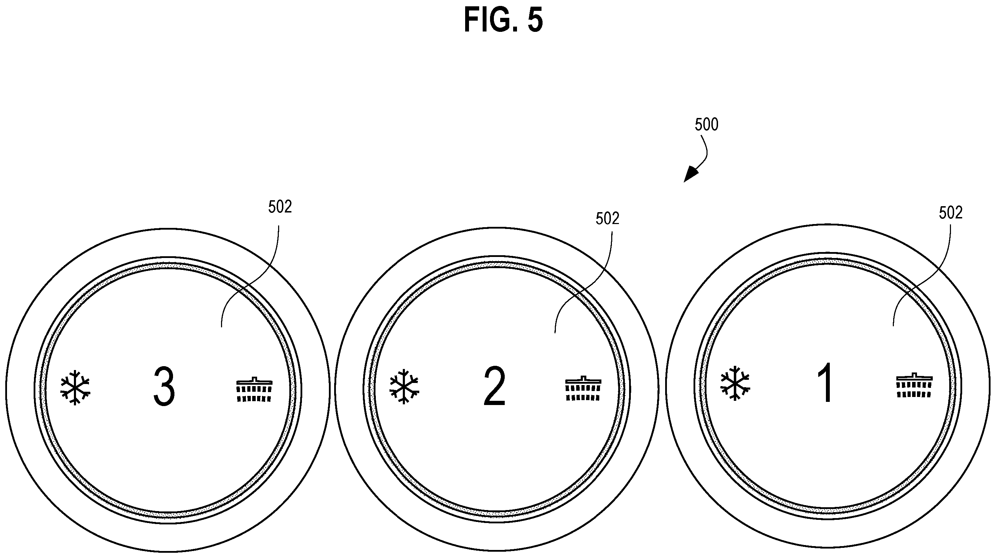

Turning to Fig. 5, a controller or UI 500 may include an optional or selectable change in display to indicate to a user when the boost therapy session will begin. The previous display of the temperature for a normal shower (illustrated in Fig. 4) may be temporarily changed, prior to indicating the temperature of the boost therapy session, to a countdown indicator 502. The countdown indicator 502 may display to a user a countdown process, such as indicating to a user "3, 2, 1" prior to initiating water flow. It should be understood that, while a variety of indicators may be used, other forms of countdowns are contemplated. Such contemplated countdowns, for example, may be a line or alternative configurations of dots or figures fading away, lights, such as LEDS integrated into controller that may fade from bright to dark or dark to bright, etc. Upon reaching the end of the countdown, the boost therapy session may commence.

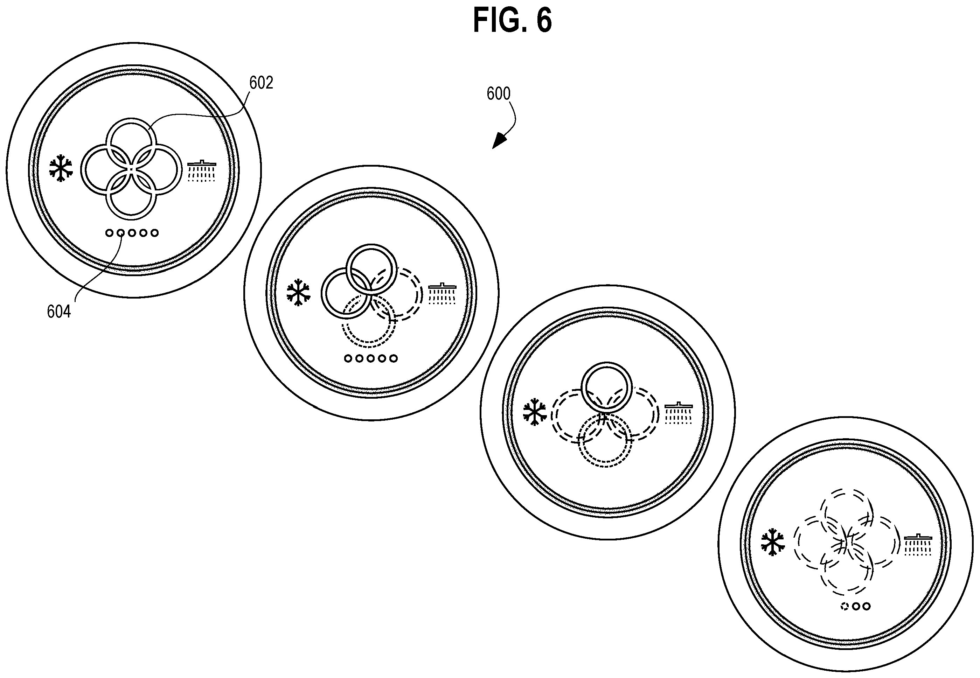

As illustrated in Fig. 6, a controller or UI 600 may include an additional, alternative, optional or selectable change in display to indicate to a user the progress of the boost therapy session. The previous display of temperature of a normal shower may be temporarily changed, prior to indicating temperature of the boost therapy session, to a countdown indicator. Following or alternative to a countdown indicator, the controller 600 may indicate the time remaining in the boost therapy session. For example, an initial state may be displayed as a beginning of the boost therapy session, such as one or more illuminated rings 602.

As depicted, the illuminated rings 602 may gradually fade away and reilluminate as the boost therapy session progresses. The illuminated rings 602 may gradually fade away or reilluminate in a clockwise, counterclockwise, or random fashion. Alternative configurations are contemplated in which the illuminated rings 602 may gradually fade away or reilluminate. The rings 602 may be beneficial to a user under the cold or hot boost therapy session, such as to provide the user with a sense of calm. The rings 602 that may fade in and out of illumination may offer the user a sense of timing to control their breathing patterns during the boost therapy sessions. For example, as one ring fades, the user may be indicated to breath in or out and vice versa. When a next ring illuminates, the process may indicate for the user to do the opposite. Once all of the illuminated rings 602 have faded away from display on the controller 600, such display will be an indication to the user that the boost therapy session has completed or is about to be completed.

A series of eliminated dots 604, as depicted, may be displayed. The illuminated dots 604 may fade away as time passes during the boost therapy session, which may indicate the time remining in the boost therapy session. Once all illuminated dots have faded away, the boost therapy session may be indicated as complete, and water flow may cease. Once all illuminated dots have faded away, the boost therapy session has completed, and a normal shower session may automatically proceed. While the illuminated rings 602 and series of illuminated dots 604 have been depicted, additional or alternative displays and illuminations are contemplated such that the symbolic nature of the illuminations ensures focus on the experience of the boost therapy sessions as opposed to the time remaining in the boost therapy session. As these boost therapy sessions may be overwhelming, quite stimulating, shocking, or seem abnormally prolonged to a user, the displays, illuminations, etc. may be depicted, displayed or illuminated in a manor to assist the user and distract the attention of the user from the boost shower therapy sessions.

While a variety of indications to a user may be used for timing and duration for a boost therapy session, other forms of countdowns are contemplated. Such contemplated countdowns, for example, may be a number of various configurations of dots or figures fading away or being illuminated, other various lights or displays (e.g., LEDs or LCDs integrated into the controller), illuminations fading from bright to dark or dark to bright, etc.

Various prompts or messages may be presented to the user, for example, before, during or after the boost therapy session. For example, during the boost therapy session, a message may be presented to the user to "Breathe Deeply," which may assist to calm, reassure, or remind the user during the boost therapy session. Following a boost therapy session, messages may also be presented to the user for reinforcement, such as a "Well Done" message. While a couple of example messages have been presented, other messages, displays, or otherwise displays to the user are contemplated.

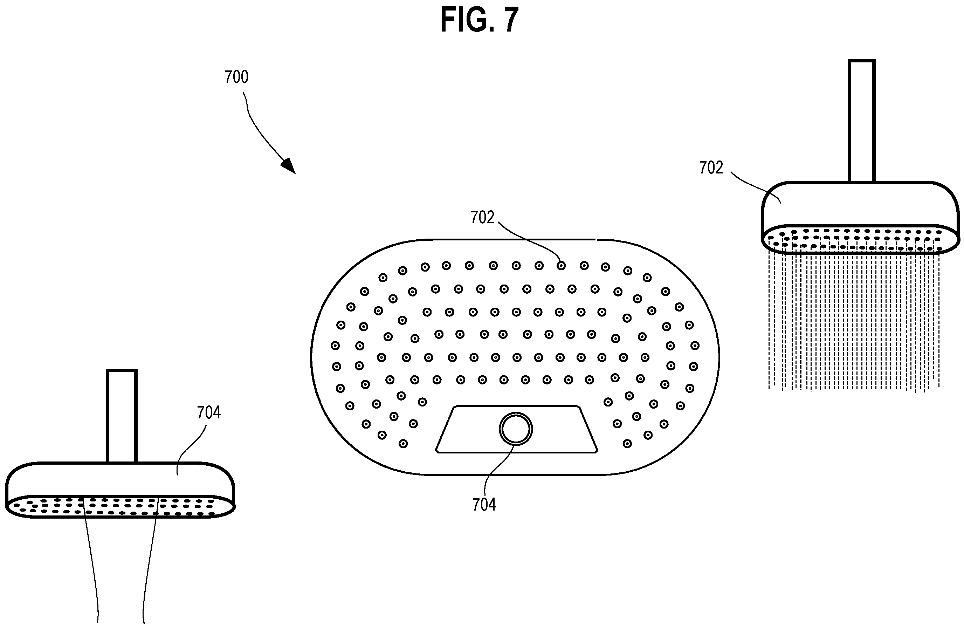

Turning to Fig. 7, a shower head 700 may include a plurality of first outlets 702 to output cold, hot, or a blend of water for conventional shower spray and use. The conventional shower spray may be modified or adjusted based on the type of shower spray head. The shower head may be rotatable, integrate a lever, or other mechanisms that may be adjusted to modify the type of spray head output, such as a mist, one or more jet streams, or alternative conventional spray as depicted as 702. The spray head 700 may include an additional secondary outlet 704 to output cold or hot water as a therapy boost session. Output from the secondary outlet 704 may include a plunge or dump type distribution of boost therapy onto a user as depicted. The cold or hot water may be pumped or otherwise provided through the secondary outlet 704 from one or more of the storage tanks, which may be temperature controlled and provided directly from one or more of the freshwater inlets, via a digital mixing valve, or other alternative means. The secondary outlet 704 may provide additional or alternative boost therapy water output, such as a mist, one or more jet streams, conventional shower spray, etc. The boost therapy shower management systems, as described herein, may alternate between conventional shower spray, cold boost therapy sessions, or hot boost therapy sessions seamlessly, even during a single use of the shower management system.

The boost therapy shower management system may include a tank purge mode. In instances where the chilled or hot storage tanks have not been used for a period of time, the stored water may be required to be or beneficial to be purged and refreshed with new, fresh water. Said period of time may be a predetermined period of time. The corresponding valves associated with the cold or hot water storage tanks may be opened to allow the stored water to be output and drained within the shower system. The drained output may be output through the corresponding, designated output of the shower head, such as being output through the cold or boost therapy session outputs. The purge or cleansing of the storage tanks may be fluidly coupled to an alternative output, such as a third or fourth output to allow for the stored water to drain out a drainage of the shower system. While examples of output have been depicted to purge and refresh water in the cold or hot storage tanks, other additional or alternative means of diverting and refreshing the water to and from the storage tanks are contemplated. Cold or hot water stored in their corresponding storage tank may be utilized and diverted as chilled or hot water for use and mixing during a normal shower. For example, chilled water stored in the chilled storage tank may be used instead of the cold freshwater inlet for use and mixing during a normal shower. The use of the cold or hot storage water may be used without the user knowing the transition. Thus, less water waste may be achieved.

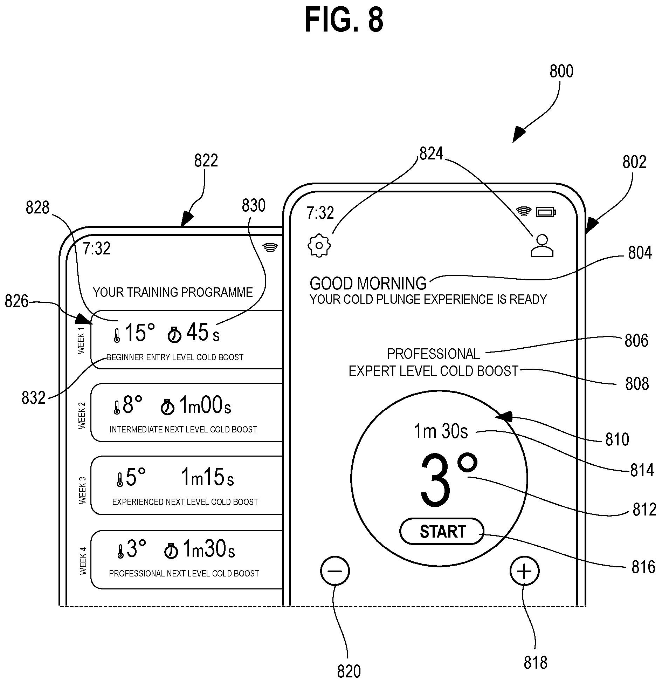

As illustrated in Fig. 8, a remote-control application 800 may be used to implement a boost therapy shower management system for cold or hot shower boost therapy sessions. A user may utilize an electronic device, such as a mobile phone, tablet, computer, or other type of device capable of communicating with a controller or other communicatively coupled systems or components integrated with a boost therapy shower management system as described herein to monitor and control boost therapy sessions. As depicted and displayed in the application, a variety of customizable, selectable, and informative features/information may be presented to a user.

For example, the application 800 may contain a first view 802. The first view 802 may comprise a plurality of features and information for presentation to a user. The first view 802 may include a greeting or message 804, a level of therapy selected 806, and/or a cold or hot level of therapy selected 808. Examples of a level of therapy selected 806 may include a beginner level, average level, professional level, extreme level, etc. for hot or cold boost therapy sessions. The first view 802 may include a section 810, which may display to a user a default or predetermined a boost therapy session for initiation. For example, a temperature 812 of the pending boost therapy may be displayed. A timer 814 may be displayed, which may indicate to a user the duration of the pending boost therapy session. Section 810 may include a start or initiate selections 816 for the user to activate the boost shower therapy session. One or more selections, for example, a positive ("+") button 818 and a negative ("-") button 820 may be integrated to provide the user with a means to dynamically adjust the temperature 812 or the timer 814 of the pending boost therapy session. In general, the first view 802 may be an initial or default view presented to the user, which may provide an overall status and control of boost shower therapy sessions.

A second or additional view 822 may be viewable to the user. The user may be redirected to second view 822 via one or more setting or personalizer selections 824 on the first view 802. The second view 822 may provide the user with the user's training program or past statistics information. For example, the second view 822 may include one or more indicators to inform the user of past performances and their boost therapy journey. It is important to note, it may be undesirable to go straight to an extreme temperature, such as going straight into "professional mode" with extended periods of extreme cold or hot temperatures. It is important to build a tolerance during hot or cold boost therapy sessions. Thus, keeping informed of past therapy sessions over the course of a period of time 826 may assist the user to develop their tolerance.

For example, the second display 822 may provide the user with information from past boost therapy sessions over the course of past weeks. Each period of time 826, for example, for each week, may include a variety of information from past boost therapy sessions. For example, each period of time 826 may display to the user the temperature of water used 828 for boost therapy sessions, a time duration 830 for the boost therapy sessions, a level of therapy 832, a "next level" preview for the next time period and temperature of a next boost therapy session, the number of boost therapy sessions experienced, and the like. It should be understood that, while exemplary information and controls are displayed in, at least in part, first view 802 and second view 822, additional or alternative features and controls are contemplated. For example, the application 800 may include alternative tracked periods of time 826, variations of displays 810, timers 814, temperature displays 816, temperature/timer controls 818 and 820, etc.

The application 800 may utilize machine learning techniques to automatically indicate for the user a next boost therapy shower session. Machine learning may take into account all, or at least portions of, past boost therapy information, such as the allotted time for each boost therapy session, temperatures of the water for each therapy session, experience level, the amount of boost therapy sessions, and the like to facilitate training programs for each user. The temperatures for each boost therapy session and timing may be tailored to each user to enable the user to build up their tolerance and experience gradually more intense or longer boost therapy sessions. The application 800 may include additional programming to enable the processing of fault diagnostics in order to detect and correct any determined faults or issues for programming and controlling application 800 and facilitating boost therapy shower sessions.

The instant application may have numerous benefits over existing solutions. The boost therapy shower management system, as described herein, may provide an integrated chilled or heated water monitoring system, which may continuously maintain or provide accurate, desired temperature for a boost therapy session. The boost therapy shower management system may provide an integrated management of a plurality of inlet supplies for different temperature ranges and optional storage thereof. A tank purge mode for cleaning cycles utilizing the stored chilled or heated supply may be mixed with cold or hot inlet water for a 'normal' shower, thus reducing water waste. The boost therapy shower management system may provide the user the ability to mix chilled or hot water to achieve a wider range of temperatures not usually achievable with cold or hot water mains inlets. Optional recirculating loops and thermistors may provide a reduction of cold or hot dead leg due to monitoring of the cold or hot recirculation loops. The boost therapy shower management system may provide a freedom to install one or more valve remotes from the chiller and storage tanks. The valve(s), chiller, and heater systems may be coupled together or be separate.

Additional or alternative features or iterations may be incorporated among variations of recirculating shower systems, as similarly described herein. For example, iterations of a recirculating shower system may include a level of improved filtration. One or more additional filters or improved filtration systems or mechanism may be integrated into a recirculating shower system. Ultraviolet disinfection (e.g., UV, UV-C, UVGI, etc.) may be incorporated into a recirculating shower system. The improved filters, Ultraviolet disinfection, or a combination thereof may be integrated inline with the plumbing. Coupling or otherwise integrating the improved filters, Ultraviolet disinfection, or a combination thereof into sump is considered. Coupling said components within the sump may provide easier access and serviceability for the user.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that may be used to store desired program code in the form of instructions or data structures and that may be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The present disclosure is disclosed in the context of shower systems but not limited to shower systems. Aspects of the present disclosure that are depicted in the illustrated embodiments or otherwise described herein may be used in conjunction with other ablutionary fittings or water distribution systems. Water distribution systems encompassed by the present disclosure include, without limitation, water distribution systems that dispense water for consumption and/or washing and water distribution systems used for private, public, domestic, residential, commercial, and/or industrial use. Water distribution systems such as, for example and without limitation, showers, baths, washtubs, hot tubs, sinks, fountains, water dispensers, and the like may incorporate aspects of the present disclosure and are encompassed herein. Example water distribution systems may include an outlet dispensing water or other fluid. The outlet may include any suitable device that may dispense liquid or water. The outlet may include an ablutionary fitting, such as, for example and without limitation, a showerhead, shower spray, wand hand shower, faucet, wand, spigot, tap, spout, or the like. The outlet can include a single outlet or more than one outlet. Where the outlet includes multiple, e.g., two or more outlets, the outlets can be similar types of outlets or dissimilar types of outlets. Elements and features described with reference to one illustrated embodiment are not limited to that embodiment only; the features and elements of any one or more of the illustrated embodiments can be utilized in any other embodiment in any combination.

As is evident from the foregoing description, certain aspects of the present invention are not limited by the particular details of the examples illustrated herein, and it is therefore contemplated that other modifications, applications, variations, or equivalents thereof, will occur to those skilled in the art. Many such changes, modifications, variations, and other uses and applications of the present constructions will, however, become apparent to those skilled in the art after considering the specification and the accompanying drawings. In addition, unless mention was made above to the contrary, it should be noted that all of the accompanying drawings are not to scale. All such changes, modifications, variations, and other uses and applications which do not depart from the spirit and scope of the present inventions are deemed to be covered by the inventions which are limited only by the claims which follow.

## Claims

1. A boost shower therapy system, comprising:
one or more inlets for receiving water from at least one of a mains water source, a stored chilled water source, or a heated water source;
an inlet management unit; and
a temperature controller for controlling at least one of the stored chilled water source and the heated water source;
wherein, upon activation via a control unit of a cold therapy session, the boost shower therapy system delivers chilled water from the stored chilled water source to a user for a first predetermined period of time; and
wherein, upon activation via the control unit of a hot therapy session, the boost shower therapy system delivers heated water from the heated water source to the user for a second predetermined period of time.

2. The boost shower therapy system of claim 1, wherein the temperature controller comprises a chiller to chill water in the stored chilled water source to a first predetermined temperature for selective output as a cold boost therapy session.

3. The boost shower therapy system of claim 1 or claim 2, wherein the temperature controller comprises at least one of a heat exchanger and heating element to heat water in the heated water source to a second predetermined temperature for selective output as a hot boost therapy session.

4. The boost shower therapy system of claim 1, claim 2 or claim 3, wherein the temperature controller comprises:
a first storage tank including a chiller to chill water in the stored chilled water source to a first predetermined temperature for selective output as a cold boost therapy session; and
a second storage tank including at least one of a heat exchanger and heating element to heat water in the heated water source to a second predetermined temperature for selective output as a hot boost therapy session;
optionally wherein a thermistor is in fluid communication with the chiller or the heat exchanger and heating element to monitor and regulate a temperature of the water.

5. The boost shower therapy system of any one of the preceding claims, wherein: the control unit comprises one or more boost shower therapy session activation selections, each including one or more boost shower therapy activation inputs, wherein the one or more boost shower therapy session activation selections include one or more of a cold boost therapy session, a hot boost therapy session, and a normal shower session; and/or the control unit includes one or more input settings to modify a temperature or an amount of the water or the first or second predetermined period of time for a next boost shower therapy session.

6. The boost shower therapy system of any one of the preceding claims, wherein an application captures and retains information from previous boost shower therapy sessions.

7. The boost shower therapy system of claim 6, wherein: the information retained by the application includes one or more of a duration of each previous boost shower therapy session, each previous temperature, and a capacity of the water used during previous boost shower therapy sessions; and/or at least one of the control unit and the application comprises a machine learning feature that retains and monitors previous boost shower therapy sessions and formulates a next or anticipated boost shower therapy session.

8. The boost shower therapy system of any one of the preceding claims, wherein the chilled or heated water is periodically circulated through a recirculating plumbing system that monitors and maintains a temperature of the water in a storage tank.

9. A boost shower therapy system comprising:
one or more inlets for receiving water from at least one of a mains water source, a stored chilled water source, or a heated water source;
a control unit for controlling operation of the boost shower therapy system;
an inlet management unit; and
a temperature controller for controlling at least one of the stored chilled water source and the heated water source, wherein the temperature controller comprises:
a first storage tank including a chiller to chill water in the stored chilled water source to a first predetermined temperature for selective output as a cold boost therapy session; and
a second storage tank including at least one of a heat exchanger and heating element to heat water in the heated water source to a second predetermined temperature for selective output as a hot boost therapy session.

10. The boost shower therapy system of claim 9, wherein: upon activation via the control unit of a cold therapy session, the boost shower therapy system delivers chilled water from the stored chilled water source to a user for a predetermined period of time; and/or upon activation via the control unit of a hot therapy session, the boost shower therapy system delivers heated water from the heated water source to a user for a predetermined period of time.

11. The boost shower therapy system of claim 9 or claim 10, wherein: the control unit comprises one or more boost shower therapy session activation selections, each including one or more boost shower therapy activation inputs, wherein the one or more boost shower therapy session activation selections include one or more of the cold boost therapy session, the hot boost therapy session, and a normal shower session; and/or the control unit includes one or more input settings to modify a temperature or an amount of the water for a next boost shower therapy session.

12. A boost shower therapy system comprising:
one or more inlets for receiving water from at least one of a mains water source, a stored chilled water source, or a heated water source;
a control unit for controlling operation of the boost shower therapy system;
an inlet management unit;
a temperature controller for controlling at least one of the stored chilled water source and the heated water source, wherein the temperature controller comprises a chiller to chill water and at least one of a heat exchanger and a heating element to heat water; and
a thermistor for monitoring and regulating a temperature of the water.

13. The boost shower therapy system of claim 12, wherein the temperature controller comprises:
a first storage tank including the chiller to chill water in the stored chilled water source to a first predetermined temperature for selective output as a cold boost therapy session; and
a second storage tank including at least one of the heat exchanger and heating element to heat water in the heated water source to a second predetermined temperature for selective output as a hot boost therapy session.

14. The boost shower therapy system of claim 12 or claim 13, wherein an application captures and retains information from previous boost shower therapy sessions.

15. The boost shower therapy system of claim 14, wherein at least one of the control unit and the application comprises a machine learning feature that retains and monitors previous boost shower therapy sessions and formulates a next or anticipated boost shower therapy session.
